(19)

Europäisches
Patentamt
European
Patent Office
Office européen
des brevets

(11) **EP 4 258 016 A1**

(12) **EUROPEAN PATENT APPLICATION**

(43) Date of publication:
**11.10.2023  Bulletin 2023/41**

(21) Application number: **22179415.9**

(22) Date of filing: **16.06.2022**

(51) International Patent Classification (IPC):
***G01S 7/52*** *(2006.01)*        ***G01S 15/89*** *(2006.01)*
***A61B 8/08*** *(2006.01)*

(52) Cooperative Patent Classification (CPC):
**G01S 15/8915; G01S 7/52028; G01S 7/52046;**
**A61B 8/5207**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority:  **07.04.2022  EP 22167119**

(71) Applicant: **Koninklijke Philips N.V.**
**5656 AG Eindhoven (NL)**

(72) Inventors:
• **VAN SLOUN, Ruud**
  **Eindhoven (NL)**
• **LUIJTEN, Wouter Marinus Benjamin**
  **Eindhoven (NL)**
• **OSSENKOPPELE, Boudewine**
  **5656AG Eindhoven (NL)**

(74) Representative: **Philips Intellectual Property &**
**Standards**
**High Tech Campus 52**
**5656 AG Eindhoven (NL)**

(54)    **A COMPUTER-IMPLEMENTED METHOD FOR BEAMFORMING OF ULTRASOUND CHANNEL**
**DATA**

(57)    The invention is directed to a computer-implemented method for beamforming of ultrasound channel data (2) to obtain a beamformed image, the method comprising the steps of receiving ultrasound channel data (2); determining an initial estimate of the beamformed image as intermediate beamformed image data; performing at least one iteration of a processing operation which comprises a data consistency step (6) followed by a prior step (8), wherein the data consistency step (6) takes as input the channel data (2) and the intermediate beamformed image data, performs at least one processing step (14, 16, 50, 56) which is designed to improve the consistency of the intermediate beamformed image data (10) with the channel data (2) and outputs updated intermediate beamformed image data (7); the prior step (8) takes as input the updated intermediate beamformed image data (7) and performs at least one processing step, which uses a prior assumption on the beamformed image data, to improve the updated intermediate beamformed image data (7) and outputs an improved updated intermediate image data (12); outputting the improved updated intermediate image data (12) as the beamformed image (20).

FIG. 1

**Description**

FIELD OF THE INVENTION

The invention relates to a computer-implemented method for beamforming of ultrasound channel data, a computer-implemented method for providing a trained algorithm, and a related computer program and system.

BACKGROUND OF THE INVENTION

Traditional ultrasound imaging methods usually use delay-and-sum (DAS) beamforming because of its low complexity and fast reconstruction time, as described in K. E. Thomenius, "Evolution of ultrasound beamformers," 1996 IEEE Ultrason. Symp. Proc., vol. 2, pp. 1615-1622, 1996. This method uses fixed, content invariant, apodization weights for the receiving channels. While its reconstruction speed allows real-time imaging, DAS beamforming does not provide optimal image contrast and resolution, because of its lack of content-adaptive array apodization. For example, backscatter from off-axis components is not adequately compensated for.

Adaptive beamforming algorithms improve on this by determining optimal content-adaptive apodization weights based on the acquired RF signals and applying them to the receiving channels. In the context of beamforming, "apodization" may be described as introducing a weighting function (the "apodization weights") when summing the channel data acquired by the transducer array. However, these content-adaptive methods are computationally more demanding and result in a significantly longer reconstruction time. They are therefore often not suitable for real-time ultrasound imaging.

A known adaptive beamforming algorithm is the minimum variance (MV) beamformer, in which the apodization weights are continuously optimized to minimize the variance of the received signals after apodization, while maintaining unity gain in the desired direction. MV beamforming methods are described e.g. in J. F. Synnevag, A. Austeng, and S. Holm, "Benefits of minimum-variance beamforming in medical ultrasound imaging," IEEE Trans. Ultrason. Ferroelectr. Freq. Control, vol. 56, no. 9, pp. 1868-1879, 2009. Although MV beamforming has shown to significantly improve resolution and contrast compared to DAS, it is also notoriously slow, relying on the computationally demanding inversion of an n x n spatial covariance matrix, having a complexity of $n^3$, where n is the number of channels. Therefore, MV beamforming is not used in real-time imaging. Another known adaptive beamforming method is Wiener beamforming, as described in C. C. Nilsen and S. Holm, "Wiener beamforming and the coherence factor in ultrasound imaging", IEEE Transactions on Ultrasonics, Ferroelectrics, and Frequency Control, vol. 57, no. 6, pp. 1329-1346, 2010.

B. Luijten et al., "Adaptive Ultrasound Beamforming Using Deep Learning," IEEE Trans Med Imaging, vol. 39, no. 12, pp. 3967-3978, 2020, describes a deep learning-based beamforming method, termed ABLE, in which content-adaptive apodization weights are calculated by an artificial neural network according to the input channel data. This is also described in WO2020/083918. This publication and the paper by B. Luijten et al. are herein incorporated by reference.

The majority of known beamforming methods does not use prior information in their signal processing at all, which limits achievable image quality. Further, the known content-adaptive beamforming methods are often prohibitively slow and too complex for real-time imaging.

OBJECT OF THE INVENTION

It is an object of this invention to provide a beamforming method which is capable of providing high image quality, while at the same time having reduced computational cost compared to known content-adaptive beamforming methods.

This object is solved by the computer-implemented method of claim 1, the computer-implemented method for providing a trained algorithm of claim 12, the computer program of claim 14 and system of claim 15.

SUMMARY OF THE INVENTION

According to an aspect of the invention, a computer-implemented method for beamforming of ultrasound channel data to obtain a beamformed image is provided. The method comprises the steps of

    a) receiving channel data acquired by an ultrasound transducer in response to an ultrasound transmission;
    b) determining an initial estimate of the beamformed image as intermediate beamformed image data;
    c) performing at least one iteration of a processing operation which comprises a data consistency step followed by a prior step, wherein
    d) the data consistency step takes as input the channel data and the intermediate beamformed image data, performs at least one processing step which is designed to improve the consistency of the intermediate beamformed image data with the channel data and outputs updated intermediate beamformed image data;

e) the prior step takes as input the updated intermediate beamformed image data and performs at least one processing step, which preferably uses a prior assumption on the beamformed image data, to improve the updated intermediate beamformed image data and outputs an improved updated intermediate image data; and

f) outputting the improved updated intermediate image data as the beamformed image.

The invention provides a beamforming method that processes channel data through at least one iteration of a data consistency step and a prior step. The data consistency step may be a step which improves the consistency of the solution, i.e., the intermediate beamformed image data, with the measurement data, i.e., the channel data. The data consistency step may be based on a gradient descent method. The data consistency (DC) step may be based on a data likelihood model. The DC step may comprise a trained algorithm, including trainable parameters, such as the parameters of a trainable artificial neural network. The prior step may include processing steps which are designed to improve the consistency of the solution, in particular the intermediate beamformed image data, with a prior belief. It may thus be a step of pushing the solution in the direction of the prior belief. The prior belief may be a known property of the expected beamformed image, for example, that it includes sparse data and/or that it includes a certain type of noise, for example, a Gaussian noise distribution. The prior step may include a proximal operator. The method may include further steps. For example, the method may include a DC step not followed by a prior step, e.g., as the last processing step.

The method for beamforming according to the invention may be considered as derived from a maximum-a-posteriori (MAP) estimator to a linear measurement model. It may allow the inclusion of prior information on the signal statistics, in particular through the prior step. The method for beamforming according to the invention may use a model-based architecture, which is inherited from proximal gradient descent. In preferable embodiments, the method may use a trained algorithm which allows for learning of said DC and/or prior steps from measurement data. In particular, beamformed images obtained from known, computationally expensive content-adaptive methods such as Minimum Variance (MV) or Wiener beamforming may be used as output training data. Other possible output training data or training targets may be images generated with the full set of channel elements, or synthetic aperture acquisitions, where the goal is to achieve this quality with less channel elements. Further possible training data may be simulations of ultrasound channel data as input training data, with the corresponding ground-truth reflectivity maps as output training data.

The beamforming method of the invention has been demonstrated to outperform the standard delay-and-sum (DAS) beamforming, but also the state-of-the-art adaptive ultrasound beamforming using deep learning (ABLE) disclosed in the above-mentioned paper by B. Luijten et al.. At the same time, the processing time for the method is comparable to the ABLE method. Accordingly, the beamforming method of the invention provides excellent image quality comparable to adaptive beamforming techniques at high frame rates, while using conventional and/or GPU-accelerated hardware.

The invention may be used in all electronic forms of coherent beamforming. The channel data may in particular be acquired with an ultrasound probe having L channels. The probe may have several transducer elements, wherein the signal acquired by one or several transducer elements may contribute to one channel. The channel data may be acquired in a broadband pulse-echo ultrasound image setting.

In particular, the channel data may be acquired using plane-wave insonification techniques or line-scanning-based insonification, but it may also be acquired with other insonification schemes. In particular, other possible applications include intravascular ultrasound (IVUS), Doppler imaging, and both two-dimensional (2D) and three-dimensional (3D) ultrasound. Extending the scope of medical imaging, the method of the invention may also be applied to channel data acquired using sensor arrays of sensors other than ultrasound, for example, acoustic arrays or radar arrays. The channel data acquired by an ultrasound transducer in response to an ultrasound transmission may therefore also be channel data acquired using other technologies such as acoustic or radar transmissions.

The channel data may be the radiofrequency (RF) signals acquired during an ultrasound examination, in particular the RF signals acquired using an array of ultrasound (US) transducer elements. The ultrasound examination may be of the human or animal body, in particular in medical imaging applications, or alternatively it may be an ultrasound examination for other purposes such as materials testing. The channel data which is subjected to the beamforming method of the invention may be the RF data as acquired, or may be derived from this data by demodulation, in particular by demodulation with the basic ultrasound frequency of the insonification. The channel data may be real-valued or complex-valued (IQ). For at least some parts of the beamforming method, the channel data may be time-of-flight corrected. This may be the case for steps that are carried out pixel-by-pixel, wherein the channel data may be time-aligned for each pixel before processing. The channel data may include as many channels as the ultrasound transducer has transducer elements, wherein the number L of channels may typically be between 16 and 1024, preferably between 32 and 512, more preferred between 128 and 256. Thus, the channel data may be represented by a tensor Y of (preferably time-aligned) input signals. For pixel-by-pixel processing, the channel data used for each pixel may be a vector y of time-aligned channel signals. The vector may have the length L, where L is the number of channels.

The initial estimate of the beamformed image is advantageously obtained by a very simple processing operation, in order to save processing power. For example, it may be a dataset containing only a single constant value, for example the value 0. In particular, every pixel of the initial estimate of the beamformed image may have the same value, for example, the value 0 or the value of 1. In an alternative embodiment, the initial estimate of the beamformed image data is determined by a delay-and-sum beamforming method, which is a simple processing step which may be performed in real time. In examples, it has been found sufficient to use a dataset in which each pixel has the value of 0 as initial estimate.

The beamforming method of the invention includes at least one iteration of a processing operation comprising a data consistency step and prior step, preferably, consisting of a data consistency step and prior step.

The DC step takes as input the channel data, which may already be the time-aligned channel data, and the intermediate beamformed image data and performs at least one processing step designed to improve the consistency of the intermediate beamformed image data with the channel data. This step may be performed pixel-by-pixel, wherein the channel data is inputted as a vector of time-aligned channel data for each pixel. The DC step may include processing steps performed by a trained algorithm, in particular an artificial neural network. The output of the DC step is termed updated intermediate beamformed image data.

This data is taken as input to the prior step, wherein the prior step, also termed proximal step, performs at least one processing step which is designed to improve the updated intermediate beamformed image data. It may use a prior assumption on the beamformed image data, for example, that the beamformed image is sparse in the image domain, or that it is sparse in the wavelet or Fourier domain. In the latter case, the prior step may include a step of transforming the updated intermediate beamformed image data into the wavelet or Fourier domain and back. The prior step may include a filtering or thresholding operation. However, in case it is not possible to describe the prior beliefs analytically for the data, the prior step may be performed by trained algorithm, in particular, an artificial neural network, which has been trained from data, as described herein. It outputs an improved updated intermediate beamformed image data.

If there are no further iterations of the processing operation, this improved updated intermediate image data is outputted as the beamformed image. If there are further iterations, the improved updated intermediate image data is taken as intermediate beamformed image data and a further iteration of the DC step, and the prior step is performed.

The processing steps described herein may be carried out pixel-by pixel, wherein each pixel has a steering vector, which may be the unity vector if the channel data used as input to that step is already time-aligned. Alternatively, a tensor of channel data may be processed together.

According to an embodiment, the processing operation includes at least one processing step which is performed by a trained algorithm, in particular a trained neural network. By using alternating data consistency and prior steps, augmented with neural networks, challenges in the estimation of signal statistics may be overcome, and the quality of beamforming is thereby improved. As demonstrated by experiments using an embodiment of the inventive beamforming method including trained neural networks, termed "neural MAP", a strong robustness and generalization beyond the training data has been demonstrated. The inventive neural MAP beamformer significantly outperforms ABLE beamforming, offering stronger contrast and higher resolution. The parameters of the trained algorithm, in particular, the neural network, can be trained/learned from training data by exploiting deep learning methods.

According to an embodiment of the invention, the data consistency step includes at least one processing step which is performed by a trained neural network. In particular the data consistency steps may take on a specific, model-based form, augmented with at least one neural network to overcome challenges in the estimation of signal statistics. This form is especially well suited for beamforming ultrasound data. In preferred embodiments, the beamforming method unrolls an iterative scheme in a feedforward neural network through a series of DC and prior steps. The resulting model-based architecture allows for training of said DC and/or prior steps from input training data and output training data, as described herein. The concept of algorithm unrolling is explained in Monga et al. "Algorithm Unrolling", IEEE Signal Processing Magazine, March 2021, pp. 18-44.

According to an embodiment of the invention, the data consistency step includes the steps of processing the channel data and the intermediate beamformed image data in a first processing step; and adding the result of the first processing step to the intermediate beamformed image data to obtain the updated intermediate beamformed image data. This processing architecture has proven advantageous, since the first processing step is designed to give an estimate of the difference between the intermediate beamformed image data and the correct image and adding that to the intermediate beamformed image data. The first processing step, which is termed $f(\cdot)$, may include or consist of a trained neural network. It may for example include a neural network comprising up to 6, preferably 2 to 4 convolutional layers and up to 3 fully-connected layers. The neural network parameters may be trained by backpropagation across the complete processing operation, and across one or several iterations.

According to an embodiment of the invention, the first processing step includes the steps of computing a residual by multiplying the intermediate beamformed image data pixel-by-pixel with a steering vector and subtracting the result from the channel data; and processing the residual and the channel data by a second processing step. In this embodiment, the data consistency step has a specific form. In particular, the first processing step computes a residual as input to a further, second processing step. The residual may be obtained by pixel-by-pixel multiplying the intermediate beamformed image data at that pixel with the steering vector of that pixel and subtracting the result from the channel data, wherein the channel data may in this case be represented by a vector of length L (L=number of channels). The steering vector may be different for each pixel in the beamformed image. If the channel data is already time-aligned, the steering vector may be a unity vector. The advantage of taking a residual as input, is that thereby the consistency with the measured data may be best evaluated. The residual and the channel data are then used as input to a second processing step, and the result of the second processing step is added to the intermediate beamformed image data to obtain the updated intermediate beamformed image data.

According to an embodiment of the invention, the method, in particular the second processing step, includes calculating a set of apodization weights from the channel data, preferably by a trained neural network, and the second processing step includes multiplying the residual by the apodization weights.

According to this embodiment, a further processor, termed $h(\cdot)$, takes the channel data and yields a set of apodization weights. The apodization weights may be content-adaptive apodization weights. The processor may include or consist of a trained artificial neural network as disclosed in WO 2020/083918 A1, which is incorporated herein by reference. The same deep learning based adaptive neural network termed ABLE is disclosed in the paper by Ben Luijten et al. By using the ABLE network, a set of content-adaptive apodization weights of good quality can be obtained, in particular for each pixel with comparatively little additional computational burden. However, by contrast to the paper by Ben Luijten et al., the content adaptive apodization weights are not used directly for beamforming. Rather, they are used in the framework of a sequence of steps to process the residual, which is obtained by multiplying the intermediate beamformed image data with the steering vector and subtracting the result from the channel data. The residual is accordingly multiplied by the apodization weights, in particular, pixel-by-pixel. According to an embodiment, the result of this multiplication is the result of the first processing step and is hence added to the intermediate beamformed image data to obtain the updated intermediate beamformed image data. The processor $h(\cdot)$ that yields the set of apodization weights may be a neural network comprising 0 to 6, preferably 2 to 4 fully-connected layers, as well as 0 to 6, preferably 2 to 4 activation layers. It may also contain up to 6 convolutional layers.

According to an embodiment of the invention, the first processing step includes a processing step which is performed by a trained neural network, wherein preferably the, or part of the second processing step is performed by said trained neural network. According to another embodiment, the second processing step, which takes as input the residual, may be performed by a trained neural network. Such neural network may comprise 0 to 6, preferably 2 to 4 fully-connected layers, as well as 0 to 6, preferably 2 to 4 activation layers. It may also contain up to 6 convolutional layers. This neural network may also be trained by backpropagation across the sequence of steps of the beamforming method of the invention.

According to an embodiment of the invention, the prior step is based on the prior assumption that the beamformed image data is sparse. It is shown to be advantageous to exploit prior information on the resulting beamformed image. Sparsity is often found in medical ultrasound imaging, and therefore it is useful to make this prior assumption. Other possible prior assumptions may be that all pixels are independent from each other, and/or that they are identically distributed, for example with Laplacian distribution.

According to an embodiment of the invention, the prior step comprises a soft-thresholding step. This is advantageous in particular when the prior belief is that the image is sparse. In soft-thresholding, all pixels having a value below the value of the threshold t minus half of the range r may be set to 0. If the pixel value S is within a range r around the threshold t, the result follows a function m (S) which may be a function which increases steadily. If the pixel value S is above the threshold plus half the range, the pixel value may be unchanged. This may be mathematically expressed as follows, wherein the original value of a pixel is S and D is the output value of the soft-thresholding filter.

$$
\begin{aligned}
D &= 0 && \text{if} && S < t - r/2 \\
D &= m\,(S) && \text{if} && t - r/2 \le S < t + r/2 \\
D &= S && \text{if} && S \ge t + r/2,
\end{aligned}
$$

wherein the function m may be, for example, a sine function, a linear function, or a polynomial function. Thereby, a smooth transition between the original and the deleted values is implemented. Values just slightly below the threshold t are not set to 0, but merely attenuated. In other embodiments, the prior step may use a different proximal operator, for example hard thresholding. It may also be a soft-thresholding in a transformed domain, for example, in the wavelet or Fourier domain. In this case, the prior step may include transforming the updated intermediate beamformed image data into this domain, e.g., the wavelet or Fourier domain, and performing a soft-thresholding operation here, and transforming back into the image domain. In other embodiments, the prior step may include or consist of further filtering operations, for example, smoothing, removing noise, or image segmentation.

According to an embodiment of the invention, the prior step includes or consists of at least one processing step which is performed by a trained algorithm. The parameters of a soft-thresholding step may also be trained from data, thus a soft-thresholding step may be such a trained algorithm. In other embodiments, the trained algorithm may be a neural network. This is useful especially if the prior belief is hard to express analytically. In this case, the prior step may still perform an operation which pushes the solution in the direction of the prior belief, but the exact nature of this operation is trained from data as explained herein. For example, the entire prior step may be replaced by a neural network, which may be trained from input and output training data. Such neural network may comprise neural network 0 to 6, preferably 2 to 4 fully-connected layers, as well as 0 to 6, preferably 2 to 4 activation layers. It may also contain up to 6 convolutional layers.

According to an embodiment of the invention, the channel data is time-of-flight corrected for each image pixel before being subjected to the processing operation. This has the advantage that no further operation such as multiplication with a steering vector needs to be done. The time-of-flight correction may be performed for each pixel of the beamformed image. The time-of-flight corrected channel data may be stored in a memory or buffer.

According to an embodiment of the invention, the method is performed by an algorithm which uses parameters which have been trained from training data. The parameters that may be trained from input and output data include the weights and biases of the one or several neural networks which may be included in the processing operation. It may also include soft-thresholding parameters, in particular, in embodiments where the prior step is includes soft-thresholding, such as the threshold, the range and the parameters of the function applied in the range.

According to an embodiment of the invention, a pre-determined number of iterations of the processing operation is carried out, preferably 1 to 20, more preferably 2 to 10 iterations. It is advantageous to determine the number of iterations beforehand, because otherwise the improved updated intermediate image data has to be examined in order to determined when to stop the algorithm, which adds to the overall processing time. Surprisingly, very few iterations have been shown to be sufficient, for example 1 to 6, preferably 2 to 4. Therefore, it has proven advantageous to simply perform a pre-determined number of iterations and then stop. If the processing operation of the method of the invention includes trainable parameters, it may be advantageous to train the algorithm with all steps and iterations together. Also in this event, a pre-determined number of iterations is advantageous. It is also possible to train the algorithm using one iteration, and then using one or several iterations when performing the beamforming method of the invention. Other embodiments include a step of processing the improved updated intermediate image data to determine whether the beamformed image is of sufficient quality to stop the algorithm, or whether another iteration is to be performed.

According to an embodiment of the invention, the training data comprises input training data comprising channel data acquired by an ultrasound transducer in response to an ultrasound transmission; and output training data comprising beamformed image data obtained from the input training data. The input training data may comprise channel data acquired by any of the above-mentioned insonification schemes, for example, plane-wave imaging. The output training data is typically obtained by a content-adaptive beamforming method, such as the minimum variance beamformer, or by Wiener beamforming. These methods provide very good results but are typically computationally too expensive to be executed in real time. However, when training the beamforming algorithm, processing time is not an issue, and therefore the beamforming method of the invention can include trainable parameters which are trained from minimum variance beamformed data. The training data may be ultrasound data acquired from human or animal subjects. It may also be simulated data, for example, simulated point scatterers from a single plane wave ultrasound acquisition.

According to a further aspect of the invention, a computer-implemented method is provided for providing a trained algorithm, which is adapted to perform the method of one of the preceding claims and which includes trainable parameters, preferably parameters of a trainable neural network, the method comprising:

(a) receiving input training data, the input training data comprising channel data acquired by an ultrasound transducer in response to an ultrasound transmission;
(b) receiving output training data, the output training data comprising beamformed image data obtained from the input training data, preferably by a content-adaptive beamforming algorithm such as a minimum variance algorithm;
(c) training the algorithm by using the input training data and the output training data;
(d) providing the trained algorithm.

The trained algorithm may have the features described in relation to the inventive beamforming method, and vice versa. In particular, it may comprise at least one trainable neural network (NN), in particular included in the data consistency step and/or the prior step. The training step may be performed using backpropagation. Thereby, the input training data, in particular the channel data, is propagated through the algorithm using predetermined initial values for the trainable parameters, in particular the weights and biases of the NN or NNs, and, where applicable, the parameters of the soft-thresholding. The output of the algorithm is compared to the output training data, i.e., the beamformed image, for example, using an error function or cost function, the output of which is propagated back through the algorithm, thereby calculating gradients to find the trainable parameters that yield minimum errors. It has been found that the parameters of the trained algorithm according to the invention converge quickly to minimum, so that the algorithm can be trained in a limited number of data relating to only one or a few ultrasound images. In some embodiments, the neural network or networks comprise dropout of layers during training. Thereby, certain parameters and the dropout layer are randomly selected, and their values are set to 0. This has the advantage that the training converges to a useful minimum.

Accordingly, using dropout layers improves the credibility of the algorithm.

According to an embodiment, the trained algorithm comprises the steps of receiving channel data acquired by an ultrasound transducer in response to an ultrasound transmission; determining an initial estimate of the beamformed image as intermediate beamformed image data; performing at least one iteration of a processing operation which comprises a data consistency step followed by a prior step, wherein the data consistency step takes as input the channel data and the intermediate beamformed image data, performs at least one processing step which is designed to improve the consistency of the intermediate beamformed image data with the channel data and outputs updated intermediate beamformed image data; the prior step takes as input the updated intermediate beamformed image data and performs at least one processing step, which preferably uses a prior assumption on the beamformed image data, to improve the updated intermediate beamformed image data and outputs an improved updated intermediate image data; and outputting the improved updated intermediate image data as the beamformed image,

wherein at least one of steps c), d) and e) includes parameters, preferably parameters of a trainable neural network, which are trained by using the input and output training data.

According to a further aspect, the invention is directed to a computer program comprising instruction and/or program code, which, when the program is executed by a computational unit, causes the computational unit to carry out a method according to an embodiment of the invention. The computer program may, in particular, carry out the method for beamforming according to the invention. It may also carry out the method for providing a trained algorithm. The computer program may be provided in the form of a computer program product. The computer program may be provided on a non-transitory digital storage medium. Such storage medium may be any optical or magnetic digital storage medium, such as a hard disk, floppy disk, DVD, CD-Rom, USB-stick, SD- card, SSD-card, etc. The storage medium may be part of a computer, server, or cloud computer. The computational unit may be any digital processing unit, in particular a CPU or GPU of a PC, server or cloud computer.

The invention is also directed to a non-tangible digital storage medium on which a computer program is stored, which comprises instructions and/or program code which, when the program is executed by a computational unit, causes the unitary carryout. A method according to an aspect or embodiment of the invention.

The beamforming method of the invention is preferably computationally so inexpensive that it can be performed at the point-of-care during an ultrasound examination of a patient. In an embodiment, it may be performed in real time, either by a computational unit which is present at the point-of-care, such as a PC, server, or a GPU or CPU, which is part of the ultrasound scanner with which the sound channel data is acquired. The channel data may also be transferred to a centralized computing unit, either in the cloud or on the server of a hospital, and the computer implemented method for beamforming may be performed on the centralized server, transferring the beamformed image back to the point-of-care. In useful embodiments, the method for beamforming may be performed in real time, so that the beamformed image is available directly after the acquisition of the channel data.

According to a further aspect, the invention is directed to a system for beamforming of ultrasound channel data to obtain a beamformed image, the system comprising a first interface, configured for receiving channel data acquired by an ultrasound transducer in response to an ultrasound transmission; a computational unit configured for determining an initial estimate of the beamformed image as intermediate beamformed image data; and performing at least one iteration of a processing operation which comprises a data consistency step followed by a prior step, wherein the data consistency step takes as input the channel data and the intermediate beamformed image data, performs at least one processing step which is designed to improve the consistency of the intermediate beamformed image data with the channel data and outputs updated intermediate beamformed image data; the prior step takes as input the updated intermediate beamformed image data and performs at least one processing step, which preferably uses a prior assumption on the beamformed image data, to improve the updated intermediate beamformed image data and outputs an improved updated intermediate image data; and a second interface, configured for outputting the improved updated intermediate image data as the beamformed image.

The system may be a digital processing unit, in particular a GPU or CPU of a computer, such as a PC, cloud computer, server, or it may be the processing unit of an ultrasound scanner.

According to a further aspect, the invention is directed an ultrasound scanner incorporating a system for beamforming of ultrasound channel data according to this invention. The ultrasound scanner may further comprise an ultrasound probe having a number L of channels, which is adapted for ultrasound insonification of a target tissue of a patient. The probe may comprise a two-dimensional or three-dimensional array of ultrasound transducer elements. Each element may correspond to an ultrasound channel. In some embodiments, several ultrasound transducer elements contribute to one single channel data.

All features described with respect to the computer-implemented method for beamforming are also applicable to the computer program, the system for beamforming, the training method and the ultrasound scanner and vice versa.

BRIEF DESCRIPTION OF THE DRAWINGS

Embodiments of the invention shall now be described with reference to the attached drawings, in which

Fig. 1    is a schematic overview of an embodiment of the inventive method;
Fig. 2    is a schematic flow diagram of an embodiment of the data consistency step;
Fig. 3    is a schematic flow diagram of an embodiment of the data consistency step;
Fig. 4    is a schematic overview of a processor designed to yield apodization weights;
Fig. 5    is a neural network, according to an embodiment;
Fig. 6    is an example of a soft-thresholding filter;
Fig. 7    is an image of simulated point scatterers, which has been beamformed according with (a) standard DAS beamformer, (b), state-of-the-art ABLE beamforming, and (c) an embodiment of the inventive beamforming algorithm, neural MAP;
Fig. 8    is a schematic view of a system according to an embodiment of the invention.

DETAILED DESCRIPTION OF EMBODIMENTS

Many classical beamforming methods can be derived from a maximum likelihood (ML) estimation p(y|x) of a narrowband linear measurement model with additive noise given by

$$y = a_\theta x + n,$$

where y is a vector of channel data, x is the tissue reflectivity, i.e., a pixel value of the target image, $a$ is a steering vector for that pixel and n additive noise. The model above assumes independent sources x (i.e., each pixel is independent), as is typically assumed in beamforming setups. In the broadband pulse-echo ultrasound imaging setting, preferably a TOF-correction / time-alignment is first performed, so that the steering vector simplifies to $a_\theta = 1$. In beamforming one aims to find the most likely candidate $\hat{x}$ that explains our measurements y. As such one aims to maximize the probability

$$p(x|y) \propto p(y|x)\, p(x),$$

which is derived from Bayes' theorem. The resulting solution requires knowledge on signal statistics. Additionally, since there is no closed form solution to this problem, this may be solved through iterative methods, such as proximal gradient descent. Without any assumptions on the prior distribution of x (p(x) = 1), the above estimator becomes the maximum-likelihood (ML) estimator of $\hat{x}$. Standard Delay-and-Sum beamforming yields the ML estimate for $\hat{x}$ under the assumption of Gaussian noise, where n ~ N(0, $\sigma^2 I$), and yields the closed form solution

$$\hat{x} = 1/L\ \mathbf{1}^T y.$$

This corresponds to summing the individual channel signals. Similarly, one can derive the ML solution for a coloured noise profile n ~ N(0, C $^{-1}$), where C is the noise covariance matrix. One can solve for $\hat{x}$ by maximizing the log-likelihood, or equivalently minimizing the negative log-likelihood:

$$\mathrm{argmax}_x \log p(\mathbf{y}|x) = \mathrm{argmin}_x (\mathbf{y} - a\mathrm{x})^{\top} (\mathbf{C}^{-1}) (\mathbf{y} - a\mathrm{x})$$

Setting the gradient over x to zero yields:

$$\nabla\mathrm{x} = -2\ \mathbf{1}^{\top} \mathbf{C}^{-1} (\mathbf{y} - \mathbf{1}\mathrm{x}) = 0$$

$$x = (\mathbf{1}^{\top} \mathbf{C}^{-1} \mathbf{1})^{-1}\ \mathbf{1}^{\top} \mathbf{C}^{-1}\ \mathbf{y} = w_{MV}^{T}\ \mathbf{y}$$

which corresponds to minimum-variance (MV) beamforming, where $w_{MV}$ comprises the MV apodization weights. When

$C = \sigma^2 \mathbf{I}$, this is equivalent to DAS. Generally speaking, the noise covariance is not known, and is estimated from data. This process is error prone and can strongly affect image quality. Similarly, one can derive a maximum-a-posteriori (MAP) beamformer, i.e., when $p(x) \neq 1$, which assumes prior knowledge on the distribution of x. One can now maximize the log-posterior given by:

$$\text{argmax}_x \log p(x|\mathbf{y}) = \text{argmin}_x (\mathbf{y} - \mathbf{1}x)^{\mathrm{T}} (\mathbf{C}^{-1})(\mathbf{y} - \mathbf{1}x) - \log p(x)$$

For a Gaussian likelihood model, the general solution is given by

$$\hat{x} = \frac{\sigma_{\hat{x}}^2}{\sigma_{\hat{x}}^2 + w_{MV}^T \, C \, w_{MV}} \, w_{MV} \, y$$

which is known as Wiener beamforming. This general case equates to a postfilter (scaling) of the MV beamformer.

For many relevant prior distributions, the MAP estimator has no closed-form solution. According to an embodiment of the invention, the MAP estimator may be obtained through an iterative method, such as proximal gradient descent. The proposed proximal gradient descent method alternates between a data-consistency (DC) step, which is based on a data likelihood model, followed by a prior (proximal) step. In the context of beamforming, each iteration k may be written as:

$$\tilde{x}_{k+1} = x_k + 2\mu \, \mathbf{1}^{\mathrm{T}} \mathbf{C}^{-1} (\mathbf{y} - \mathbf{1}x_k)$$

$$x_{k+1} = \text{Prox} \, (\tilde{x}_{k+1})$$

where Prox($\cdot$) denotes the proximal operator operating on the beamformed image domain, C is the noise covariance matrix and $\mu$ is a gradient step size of the DC update for a multivariate Gaussian channel noise process. Like in MV and Wiener beamforming, direct implementation of the above requires estimation of the signal statistics for each pixel, i.e., the inverse covariance matrix $C^{-1}$. According to embodiments of the present invention, this slow and often instable procedure may be circumvented by learning parts of the DC step with neural networks. For simple i.i.d. (independent and identically distributed) prior distributions such the Laplacian, the proximal step may be a soft-thresholding operation. To model the proximal operators of more complex/rich prior distributions, neural networks may also be used according to an embodiment.

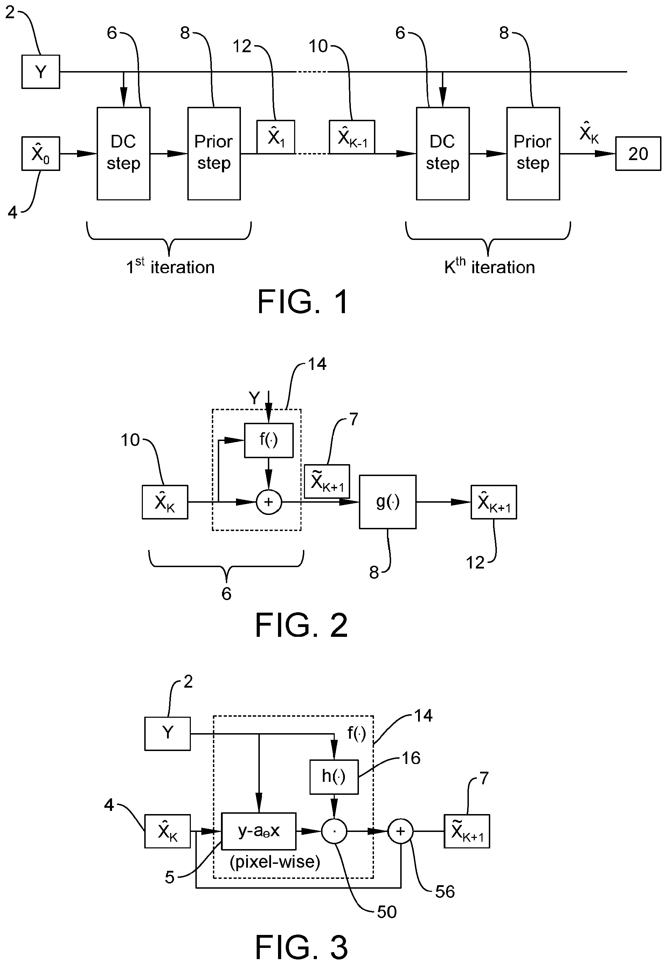

Fig. 1 shows an embodiment of the inventive beamformer for ultrasound data that processes channel signals through a series of data consistency ("DC") and "prior" steps. The beamformer has the form: $X = f(\mathbf{Y})$, where Y is a tensor of (time-aligned) input channel signals (real-valued or IQ) and X is a processed (beamformed) output image. Also, denote for each pixel a steering vector $\mathbf{a}_\theta$ with $\mathbf{a}_\theta = 1$ for time-aligned signals. Further, denote $\hat{\mathbf{X}}_0$ as an initial estimate. Accordingly, the channel data 2 as well as an initial estimate of the beamformed image 4 is the input to the DC step 6. The initial estimate $\hat{\mathbf{X}}_0$ may be an image of zeros, i.e., Xo = 0. In an alternate embodiment Xo, is a beamformed image by any other prior art beamforming method (e.g., delay-and-sum). The channel data may in particular be time-aligned for each pixel. The output of the DC step 6 is input into the prior step 8, which outputs an improved updated intermediate image data 12, denoted as $\hat{\mathbf{X}}_1$. These steps may be performed pixel-by-pixel, wherein one or several or all pixels may be processed at the same time. This updated intermediate image data 12 may be used as input 10 to a next iteration of the DC step 6 and the prior step 8. Each iteration takes as input an intermediate beamformed image data $\hat{\mathbf{X}}_{k-1}$ and gives as output improved updated intermediate image data $\hat{\mathbf{X}}_k$. According to an embodiment, a fixed number k of iterations is performed, for example, 2 to 5, more preferred 3 to 4. The processing method and the processing parameters of the DC step 6 and the prior step 8 may be the same in each iteration. In some embodiments, the processing method is the same, but the parameters are different in each iteration. In an embodiment, only the DC step comprises a trained artificial neural network, which may be the same in each iteration. In an embodiment, the prior step may consist of or comprise a soft-thresholding filter which may also be the same in each iteration. The improved updated intermediate image data which is outputted from the last iteration of the prior step 8 is then the final beamformed image 20.

Fig. 2 shows an embodiment of the DC step 6 followed by the prior step g($\cdot$), wherein only one iteration is shown. Each iteration takes as inputs the measured channel data Y obtained with an ultrasound probe having L channels, and an intermediate solution 10, denoted $\hat{\mathbf{X}}_k$. In a first processing step 14, denoted as processor f($\cdot$), these inputs are processed. The output of processor $f(\cdot)$ is added to input $X_k$ to obtain $\tilde{\mathbf{X}}_{k+1}$, such that we have:

$$\tilde{\mathbf{X}}_{k+1} = \hat{\mathbf{X}}_k + f(\mathbf{y}, \hat{\mathbf{X}}_k).$$

$\tilde{\mathbf{X}}_{k+1}$ is the updated intermediate beamformed image data 7, the output of the DC step 6. A second processor $g(\cdot)$, corresponding to the prior step 8, further processes $\tilde{\mathbf{X}}_{k+1}$ to yield the improved updated intermediate image data $\hat{\mathbf{X}}_{k+1}$, such that we have: $\hat{\mathbf{X}}_{k+1} = g(\tilde{\mathbf{X}}_{k+1})$. The processors f $(\cdot)$ and $g(\cdot)$ can be (but are not limited to) neural networks. The neural network parameters may be trained by backpropagation across the sequence of steps. The neural network parameters per step may be the same or different. The processor $g(\cdot)$ can alternatively be a soft-thresholding function. The latter is useful for imposing a sparsity prior.

In an embodiment, the first processing step 14, here denoted as processor $f(\cdot)$, takes as an explicit input a residual 5. This residual 5 may be obtained by per-pixel multiplying the intermediate solution 4 ($\hat{\mathbf{X}}_k$) at that pixel with the steering vector of that pixel $\mathbf{a}_\theta$ and subtracting that from the channel data Y, using computed steering vector $\mathbf{a}_\theta$ of length L for each pixel. In the case of time-aligned input channel data, $\mathbf{a}_\theta = 1$. For each pixel, this may be written as:

$$\tilde{\mathbf{x}}_{k+1} = f(\mathbf{y}, \mathbf{y} - \mathbf{a}_\theta \mathbf{x}_k) + \hat{\mathbf{x}}_k$$

In a specific case of the previous embodiment, which is illustrated in Fig. 3, $f(\cdot)$ may be further composed of a second processing step 16, here denoted as a processor $h(\cdot)$, that takes the channel data Y and yields a set of weights. The set of weights may be calculated only once, stored in a buffer, and used in several iterations. Processor $h(\cdot)$ may be a neural network. It may be a neural network as disclosed in WO 2020/083918 A1. In an embodiment, the sequence consists of 4 steps, and said neural network comprises 3 convolutional layers.

The second processing step further includes multiplying 50 of the residual 5 and the set of weights calculated by the processor 16 with each other element-wise. The result is added to the intermediate beamformed image data $\hat{\mathbf{X}}_k$ in step 56 to give the updated intermediate beamformed image data $\tilde{\mathbf{X}}_{k+1}$. In a per pixel implementation, this may be written as:

$$\tilde{\mathbf{x}}_{k+1} = h(\mathbf{y}) * (\mathbf{y} - \mathbf{a}_\theta \mathbf{x}_k) + \hat{\mathbf{x}}_k$$

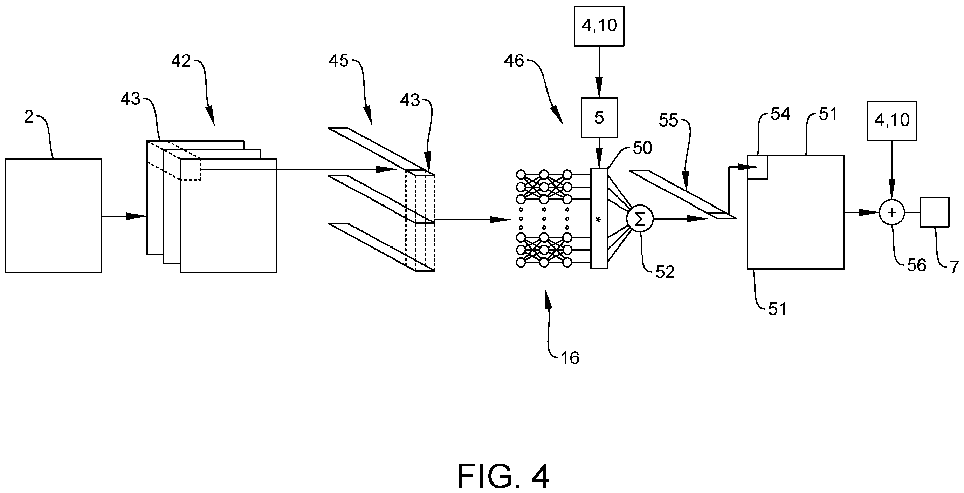

Fig. 4 shows a schematic overview of the DC step 6 according to an embodiment. The channel data, which may be RF data, are illustrated as input data 2. In step 42, the channel data is time-aligned, wherein the different planes 43 stand for the data of the different channels. The time-of-flight corrected RF signals 43 may be calculated beforehand and stored in a buffer, or alternatively computed each time it is needed by the algorithm, thereby reducing memory overhead. In this embodiment, all data 43 from the various channels relating to one pixel is rearranged into a new format 45, so that the data 43 for each pixel may be processed as a single batch in the neural network 16. The NN 16 may be as described herein below or may be different. It outputs a set of apodization weights, which are multiplied with the residual 5 in step 50. The residual 5 may be calculated as described above. The result of the multiplication 50 and weighted summation 52 is the beamformed residual 55 relating to one pixel 54. This may be used to reconstruct a beamformed residual image, 51, which is added in step 56 to the same intermediate beamformed image data 4 that is used to calculate the residual 5. The output 7 is an updated intermediate beamformed image data.

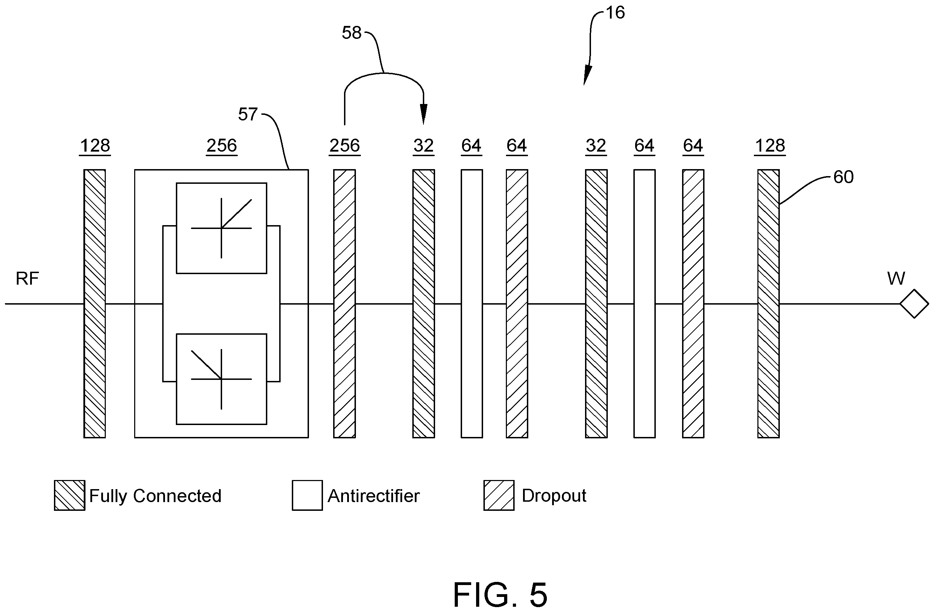

An embodiment of an artificial neural network (NN) 16 which may be used to calculate adaptive apodization weights is shown in more detail in Fig. 5. It comprises at least one fully-connected layer and at least one activation layer including an activation function which propagates both positive and negative input values with unbounded output values. By "unbounded" it is meant that the output value of the activation function is not limited to any particular value (such as +1 or -1). Preferably, any value may in principle be obtained, thereby preserving the dynamic range of the channel data. An example of such a function may be the Antirectifier function or the Concatenated Rectifier Linear Unit (CReLU), as described in the article by Shang et al., "Understanding and Improving Convolutional Neural Networks via Concatenated Rectifier Linear Unit", Proceedings of the 33rd International Conference on Machine Learning, New York, USA, 2016. Above each layer, its output size (for 128 contributing channels) is indicated. Fully-connected layers are illustrated by a dark shading, Antirectifier layers are illustrated in white, and drop-out layers (which are only present during training of the network) are illustrated in a light shading. This NN 16 comprises four fully-connected layers comprising 128 nodes for the input layer and output layer, and 32 nodes for the inner layers. This dimensionality reduction 58 by a factor of 8 ($2^3$) following the first Antirectifier layer, or by a factor of 4 with respect to the input layer, forces the network to find a more compact representation of the data. Each of the fully-connected layers (except the last layer) is followed by an Antirectifier layer. The last fully-connected layer 60 is either the output layer or is directly connected to an output layer (not shown). During training, dropout may be applied between each pair of fully-connected layers, for example with a probability of 0.2. In other words, during training a fixed percentage of the nodes in the dropout layers are dropped out.

Thus, the dropout layers are present only during training the network. The dropout helps to reduce overfitting of the neural network to the training data. The result of the NN is a set of apodization weights W.

The NN may be implemented in Python using the Keras API with a TensorFlow (Google, CA, USA) backend. For training the Adam optimizer may be used with a learning rate of 0.001, stochastically optimizing across a batch of pixels belonging to a single image. The neural network shown in Fig. 5 may be trained on *in vivo* ultrasound image data as input training data. As output training data, the apodization weights calculated by a known adaptive beamforming technique using traditional algorithms may be used. The input training data is the corresponding time-aligned RF signals / channel data. Alternatively, the neural network of the second processing step 16 may be trained together with the complete sequence of the steps of the processing operation according to an embodiment of the invention.

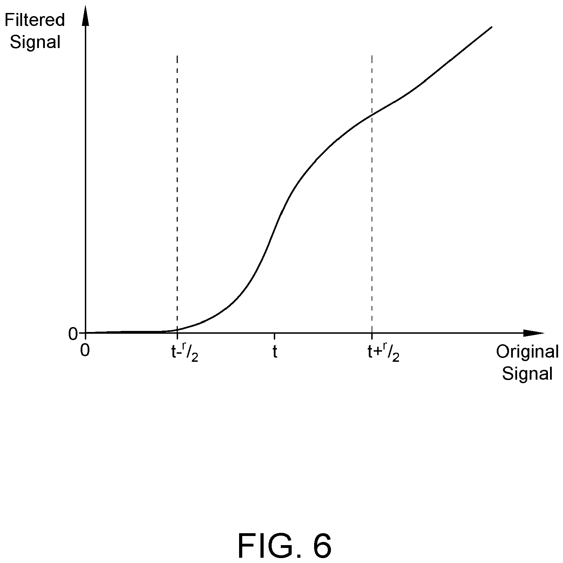

Fig. 6 illustrates a soft-thresholding filter, wherein the filtered signal is given against the original signal for values above 0. Accordingly, all signal values below the threshold t - r/2 are set to 0, where r is the range. In the interval between t - r/2 and t + r/2, the filtered signal follows a sine function. Above t + r/2, the filter signal is equal to the original signal. It may also be slightly smaller than the original signal by a predetermined value. By using this function as the prior step g, the image may be pushed towards the prior belief that the ultrasound image is sparse.

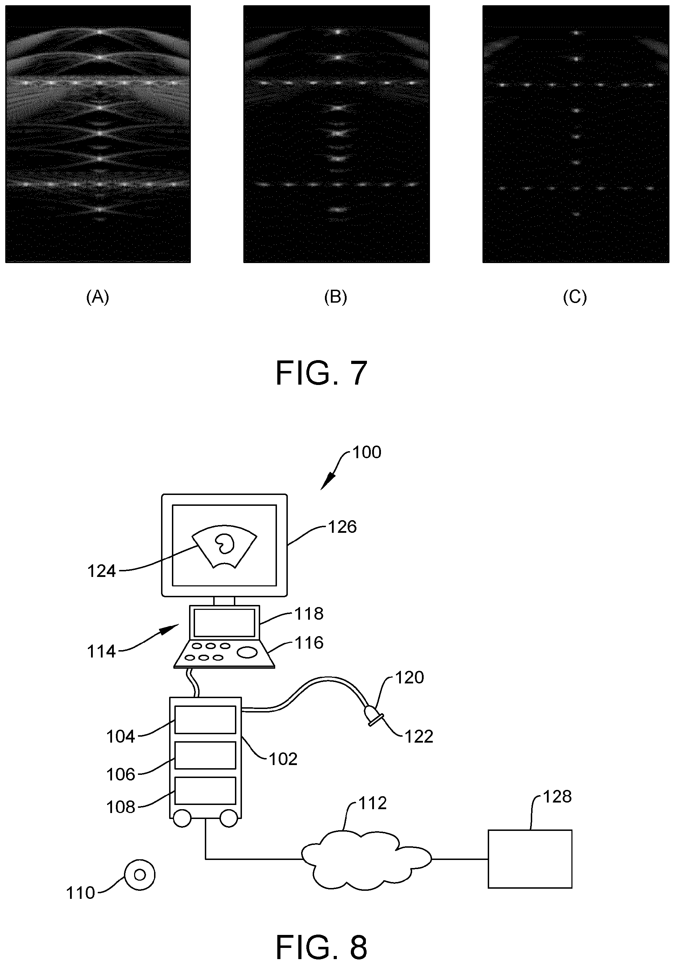

Fig. 7 shows a comparison of the image quality of simulated point scatterers from a single plane wave acquisition. The three images show the standard delay-and-sum beamforming in Fig. 7(a), the current state-of-the-art ABLE beamforming in Fig. 7(b), and the inventive neural MAP beamforming in Fig. 7(c). The neural MAP beamformer of Fig. 7(c) uses an embodiment according to Fig. 3, wherein the prior step is a soft-thresholding function, h is a neural network of 4 convolutional layers, and 4 iterations are being used. Both deep learning-based beamformers ABLE and neural MAP, were trained towards high-quality *in vivo* targets generated with 11 plane wave acquisitions using a minimum variance beamformer. Fig. 7 shows inference results on simulated data, which is vastly different from the training set, to demonstrate the strong generalization and robustness beyond the training domain. The neural MAP beamformer moreover significantly outperforms ABLE, offering stronger contrast and higher resolution.

Fig. 8 is a schematic representation of an ultrasound system 100 according to an embodiment of the invention and configured to perform the inventive method. The ultrasound system 100 includes a usual ultrasound hardware unit 102, comprising a CPU 104, GPU 106 and digital storage medium 108, for example a hard disc or solid-state disc. A computer program may be loaded into the hardware unit, from CD-ROM 110 or over the internet 112. The hardware unit 102 is connected to a user-interface 114, which comprises a keyboard 116 and optionally a touchpad 118. The touchpad 118 may also act as a display device for displaying imaging parameters. The hardware unit 102 is connected to the ultrasound probe 120, which includes an array of ultrasound transducers 122, which allows the acquisition of live ultrasound images from a subject or patient (not shown). The live images 124, acquired with the ultrasound probe 120 and beamformed according to the inventive method performed by the CPU 104 and/or GPU, are displayed on screen 126, which may be any commercially available display unit, e.g., a screen, television set, flat screen, projector etc.

Further, there may be a connection to a remote computer or server 128, for example via the internet 112. The method according to the invention may be performed by CPU 104 or GPU 106 of the hardware unit 102 but may also be performed by a processor of the remote server 128.


REFERENCE SIGNS

| | |
|---|---|
| 2 | channel data |
| 4 | initial estimate of the beamformed image |
| 5 | residual |
| 6 | data consistency step |
| 7 | updated intermediate beamformed image data |
| 8 | prior step |
| 10 | intermediate beamformed image data |
| 12 | improved updated intermediate beamformed image data |
| 14 | first processing step |
| 16 | second processing step, neural network |
| 42 | time-of-flight correction |
| 43 | time-of-flight corrected channel data |
| 45 | rearranged TOF corrected channel data |
| 50 | multiplication |
| 51 | beamformed residual image |
| 52 | summation |
| 54 | pixel |

55     beamformed residual
56     addition
57     antirectifier
58     dimensionality reduction
60     fully-connected layer
100   ultrasound system
102   hardware unit
104   CPU
106   GPU
108   digital storage medium
110   CD-ROM
112   internet
114   user interface
116   keyboard
118   touchpad
120   ultrasound probe
122   array of ultrasound transducers
124   images
126   screen
128   computer

**Claims**

1. A computer-implemented method for beamforming of ultrasound channel data (2) to obtain a beamformed image (20), the method comprising the steps of

   a) receiving channel data (2) acquired by an ultrasound transducer (122) in response to an ultrasound transmission;
   b) determining an initial estimate (4) of the beamformed image as intermediate beamformed image data (10);
   c) performing at least one iteration of a processing operation which comprises a data consistency step (6) followed by a prior step (8), wherein
   d) the data consistency step (6) takes as input the channel data (2) and the intermediate beamformed image data (10), performs at least one processing step (14, 16, 50, 56) which is designed to improve the consistency of the intermediate beamformed image data with the channel data (2) and outputs updated intermediate beamformed image data (7);
   e) the prior step (8) takes as input the updated intermediate beamformed image data (7) and performs at least one processing step, which preferably uses a prior assumption on the beamformed image data, to improve the updated intermediate beamformed image data (7) and outputs an improved updated intermediate image data (12);
   f) outputting the improved updated intermediate image data (12) as the beamformed image (20).

2. The method of claim 1, wherein the data consistency step (6) includes at least one processing step (16), which is performed by a trained neural network.

3. The method of claim 1 or 2, wherein the data consistency step (6) includes the steps of

   d.1) processing the channel data (2) and the intermediate beamformed image data (10) in a first processing step (14); and
   d.2) adding (56) the result of the first processing step (14) to the intermediate beamformed image data (10) to obtain the updated intermediate beamformed image data (7).

4. The method of one of the preceding claims, wherein the first processing step (14) includes the steps of

   - computing a residual (5) by multiplying the intermediate beamformed image data (10) pixel-by-pixel with a steering vector and subtracting the result from the channel data (2);
   - processing the residual (5) and the channel data (2) by a second processing step (16, 50).

**5.** The method of claim 4, wherein

- the method includes calculating a set of apodization weights from the channel data (2), preferably by a trained neural network (16), and
- wherein the second processing step includes multiplying (50) the residual by the apodization weights.

**6.** The method of one of claims 3 to 5, wherein the first processing step (14) includes a processing step which is performed by a trained neural network, wherein preferably the second processing step is performed by said trained neural network (16).

**7.** The method of one of the preceding claims, wherein the prior step (8) is based on the prior assumption that the beamformed image (20) is sparse.

**8.** The method of one of the preceding claims, wherein the prior step (8) includes at least one processing step which is performed by a trained neural network.

**9.** The method of one of the preceding claims, wherein the prior step (8) comprises a soft-thresholding step.

**10.** The method of one of the preceding claims, wherein a pre-determined number of iterations of the processing operation is carried out, preferably 1 to 20, more preferably 2 to 10 iterations.

**11.** The method of one of the preceding claims, wherein the method is performed by an algorithm which uses parameters which have been trained from training data.

**12.** A computer-implemented method for providing a trained algorithm, which is adapted to perform the method of one of the preceding claims and which includes trainable parameters, preferably parameters of a trainable neural network, the method comprising:

(a) receiving input training data, the input training data comprising channel data (2) acquired by an ultrasound transducer (122) in response to an ultrasound transmission;
(b) receiving output training data, the output training data comprising beamformed image data obtained from the input training data, preferably by a content-adaptive beamforming algorithm such as a minimum variance algorithm;
(c) training the algorithm by using the input training data and the output training data;
(d) providing the trained algorithm.

**13.** The computer-implemented method of claim 12, wherein the trained algorithm comprises the steps of:

a) receiving channel data (2) acquired by an ultrasound transducer in response to an ultrasound transmission;
b) determining an initial estimate (4) of the beamformed image as intermediate beamformed image data (10);
c) performing at least one iteration of a processing operation which comprises a data consistency step (6) followed by a prior step (8), wherein
d) the data consistency step (6) takes as input the channel data (2) and the intermediate beamformed image data (10), performs at least one processing step which is designed to improve the consistency of the intermediate beamformed image data with the channel data (2) and outputs updated intermediate beamformed image data (7);
e) the prior step (8) takes as input the updated intermediate beamformed image data (7) and performs at least one processing step, which preferably uses a prior assumption on the beamformed image data, to improve the updated intermediate beamformed image data and outputs an improved updated intermediate image data (12);
f) outputting the improved updated intermediate image data (12) as the beamformed image (20),

and wherein at least one of steps c), d) and e) includes parameters, preferably parameters of a trainable neural network, which are trained by using the input and output training data.

**14.** A computer program comprising instruction, which, when the program is executed by a computational unit, causes the computational unit to carry out a method according to any one of claims 1 to 13.

**15.** A system for beamforming of ultrasound channel data (2) to obtain a beamformed image, the system comprising

- a first interface, configured for receiving channel data (2) acquired by an ultrasound transducer (122) in response to an ultrasound transmission;
- a computational unit configured for

determining an initial estimate (4) of the beamformed image as intermediate beamformed image data; and performing at least one iteration of a processing operation which comprises a data consistency step (6) followed by a prior step (8), wherein

- the data consistency step (6) takes as input the channel data (2) and the intermediate beamformed image data (10), performs at least one processing step (14, 16, 50, 56) which is designed to improve the consistency of the intermediate beamformed image (10) data with the channel data (2) and outputs updated intermediate beamformed image data (7);
- the prior step (8) takes as input the updated intermediate beamformed image data (7) and performs at least one processing step, which preferably uses a prior assumption on the beamformed image data, to improve the updated intermediate beamformed image (7) data and outputs an improved updated intermediate image data (12);
- a second interface, configured for outputting the improved updated intermediate image data (12) as the beamformed image (20).

FIG. 1

FIG. 2

FIG. 3

FIG. 4

FIG. 5

FIG. 6

(A)                    (B)                    (C)

# FIG. 7

# FIG. 8

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YUELONG LI ET AL: "Deep Algorithm Unrolling for Biomedical Imaging", ARXIV.ORG, CORNELL UNIVERSITY LIBRARY, 201 OLIN LIBRARY CORNELL UNIVERSITY ITHACA, NY 14853, 15 August 2021 (2021-08-15), XP091035587, * figures 1.7, 1.8 * * chapter 1.3.3 * | 1-15 | INV. G01S7/52 G01S15/89  ADD. A61B8/08 |
| A | JINXI XIANG ET AL: "FISTA-Net: Learning A Fast Iterative Shrinkage Thresholding Network for Inverse Problems in Imaging", ARXIV.ORG, CORNELL UNIVERSITY LIBRARY, 201 OLIN LIBRARY CORNELL UNIVERSITY ITHACA, NY 14853, 25 January 2021 (2021-01-25), XP081865563, DOI: 10.1109/TMI.2021.3054167 * abstract; figure 2 * * chapter III.A * | 8 | |

| TECHNICAL FIELDS SEARCHED (IPC) |
|---|
| G01S A61B |

The present search report has been drawn up for all claims

| Place of search | Date of completion of the search | Examiner |
|---|---|---|
| Munich | 22 November 2022 | Knoll, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020083918 A

- WO 2020083918 A1

### Non-patent literature cited in the description

- **K. E. THOMENIUS.** Evolution of ultrasound beamformers. *1996 IEEE Ultrason. Symp. Proc.,* 1996, vol. 2, 1615-1622
- **J. F. SYNNEVAG ; A. AUSTENG ; S. HOLM.** Benefits of minimum-variance beamforming in medical ultrasound imaging. *IEEE Trans. Ultrason. Ferroelectr. Freq. Control,* 2009, vol. 56 (9), 1868-1879
- **C. C. NILSEN ; S. HOLM.** Wiener beamforming and the coherence factor in ultrasound imaging. *IEEE Transactions on Ultrasonics, Ferroelectrics, and Frequency Control,* 2010, vol. 57 (6), 1329-1346

- **B. LUIJTEN et al.** Adaptive Ultrasound Beamforming Using Deep Learning. *IEEE Trans Med Imaging,* 2020, vol. 39 (12), 3967-3978
- **MONGA et al.** Algorithm Unrolling. *IEEE Signal Processing Magazine,* March 2021, 18-44
- **SHANG et al.** Understanding and Improving Convolutional Neural Networks via Concatenated Rectifier Linear Unit. *Proceedings of the 33rd International Conference on Machine Learning, New York, USA,* 2016